# EUROPEAN PATENT APPLICATION

(11) **EP 0 870 835 A1**
(43) Date of publication of application: **14.10.1998**
(21) Application number: 97201022.7
(22) Date of filing: 07.04.1997
(51) Int. Cl.: C12N 15/80, C12N 1/15

(54) **Agrobacterium mediated transformation of moulds, in particular those belonging to the genus Aspergillus**

(71) Applicant: UNILEVER N.V., 3000 DK Rotterdam (NL)
(72) Inventor: The designation of the inventor has not yet been filed
(74) Representative: van der Toorren, Johannes, Drs.

(57) **Abstract**

The invention relates to *Agrobacterium* mediated transformation of moulds comprising species of the fungal sub-divisions *Ascomycotina*, *Basidiomycotina*, *Deuteromycotina*, *Mastigomycotina*, and *Zygomycotina*.
Examples demonstrate the transformation of *Aspergillus awamori* (both protoplasts and conidia), *Aspergillus nidulans*, *Aspergillus niger, Colletotrichum gloeosporioides*, *Fusarium solani pisi*, *Neurospora crassa*, *Trichoderma reesei*, and *Pleurotus ostreatus* (all conidia), and *Fusarium graminearum* (both conidia and rehydrated freeze dried ATCC material). Especially for *Aspergillus awamori* the transformation frequency is much higher than with conventional mould transformation techniques.

Information on a deposit of a micro-organism under the Budapest Treaty is given above on page 16, lines 26-29. In agreement with Rule 28 (4) EPC, or a similar arrangement for a State not being a Contracting State of the EPC, it is hereby requested that a sample of such deposit, when requested, will be submitted to an expert only.

## Description

The invention relates to the transformation of moulds, especially of moulds belonging to the genus *Aspergillus*.

### Background of the invention and prior art

### (1) General transformation techniques for micro-organisms

In recombinant DNA technology, transformation techniques for bacteria and yeasts are well developed, but transformation frequencies for moulds are relatively low.

For example, in genetic transformation of the bacterium *Escherichia coli* transformation frequencies of about 5 x 10⁸ transformants/µg vector DNA have been obtained routinely, using a chemical transformation method. Approximately 3.5% of the viable cells became transformed (Hanahan; J. Mol. Biol. 166 (1983) 557-580). More recently, even higher frequencies, of 10⁹ to 10¹⁰ transformants/µg vector DNA, have been reported after high voltage electroporation (Dower *et al*.; Nucleic Acids Research 16 (1988) 6127-6145). For other bacteria lower transformation frequencies have been described (e.g. Chassy and Flickinger; FEMS Microbiology Letters 44 (1987) 173-177; Miller *et al*.; Proc. Natl. Acad. Sci. USA 85 (1988) 856-860). For yeasts, transformation frequencies of up to 1 x 10⁷ transformants per µg vector DNA have been obtained (Meilhoc *et al*.; Bio/Technology 8 (1990) 223-227 and Giets *et al*.; Yeast 11 (1995) 355-360).
For moulds, transformation frequencies vary from
- only 0.1-0.5 transformants/µg vector DNA for *Agaricus bisporus* (Van Rhee *et al*.; Mol Gen Genet 250 (1996) 252-258), via
- 5 transformants/µg vector DNA for *Fusarium graminearum* A3/5 (Royer *et al*.; Bio/Technology 13 (1995) 1479-1483),
- about 12 transformants/µg vector DNA for *Aspergillus awamori* (Ward *et al*.; Experimental Mycology 13 (1989) 289-293), and
- 20-300 transformants/µg vector DNA for *Aspergillus nidulans* (Yelton *et al*. Proc. Natl. Acad. Sci. USA 81 (1984) 1470-1474) to
- about 10⁴ transformants/µg vector DNA for *Neurospora crassa* (Volmer and Yanofsky; Proc. Natl. Acad. Sci. USA 83 (1986) 4869-4873).

For review articles on the transformation of moulds reference is made to the articles:
- "Transformation in Fungi" by John R.S. Fincham published in Microbiological Reviews (Mar. 1989) 148-170, which gives an outline of the possible transformation methods for fungi, i.e. both yeasts and moulds.
- "Genetic engineering of filamentous fungi" by Timberlake, W.E. and Marshall, M.A. Science 244 (1989) 1313-1317.
- "Transformation" by David B. Finkelstein (Chapter 6 in the book "Biotechnology of Filamentous Fungi, Technology and Products" (1992) 113-156, edited by Finkelstein and Ball).
From this literature it is clear that several transformation techniques have been developed to transform an increasing number of filamentous fungi. Most transformation protocols make use of protoplasts. Protoplasts can be prepared from hyphal cultures or germinating conidia using Novozyme 234^{R}, a multi-enzyme preparation derived from *Trichoderma reesei*. Transformation of protoplasts with DNA is mediated by electroporation or by a combination of CaCl₂ and polyethylene glycol (PEG). Some alternative methods avoid the need for making protoplasts, which renders the procedure more rapid and simpler. Intact cells can be transformed using a combination of lithium acetate and PEG, particle bombardment (Lorito *et al*.; Curr. Genet. 24 (1993) 349-356 and Herzog *et al*.; Appl. Microbiol. Biotechnol. 45 (1996) 333-337) or also electroporation (Ozeki *et al*.; Biosci. Biotech. Biochem. 58 (1994) 2224-2227).
**In view of the relatively low transformation frequencies of moulds in relation to the transformation frequencies of bacteria and yeasts, a need exists for higher transformation frequencies in moulds.**

### (2) Plant transformation using Agrobacterium

Another transformation technique developed for plants is based on the use of *Agrobacterium tumefaciens*, which is a gram-negative soil bacterium that causes crown gall tumors at wound sites of infected dicotyledonous plants. During tumor induction *Agrobacterium* attaches to plant cells and then transfers part of its tumor-inducing (Ti) plasmid, the transferred DNA or T-DNA, to the cell where it becomes integrated in the plant nuclear genome. The T-DNA is flanked by 24 basepair imperfect direct repeats. These direct repeats are also known as "border repeats" or "borders" or "T-DNA borders" or "border sequences" or combinations thereof. The T-DNA contains a set of genes. Expression of a subset of these genes, the onc genes, leads to the production of phytohormones which induce plant cell proliferation and the formation of a tumor. The process of transfer depends on the induction of a set of virulence genes encoded by the Ti plasmid. The transfer system is activated when VirA senses inducing compounds from wounded plants, such as acetosyringone (AS). Via the transcriptional activator VirG, the remaining *vir* loci are activated and a linear single-stranded DNA, the T-strand, is produced following nicking of the border repeats by a *virD1/D2* encoded site-specific endonuclease. The VirD2 protein remains covalently attached to the 5' terminus. The T-strand is coated by the single-strand binding protein VirE and the resulting complex is transferred to the plant cell. Although the mechanism by which the T-DNA complex is transported from the bacterium into the plant cell is not well understood, it is thought that the T-DNA complex leaves the *Agrobacterium* cell through a transmembrane structure consisting of proteins-encoded by the *virB* operon. For extensive reviews on *Agrobacterium tumefaciens* transformation see Hooykaas and Schilperoort (Plant Molecular Biology 19 (1992) 15-38) and Hooykaas and Beijersbergen (Annu. Rev. Phytopathol. 32 (1994) 157-179). The ability of *Agrobacterium tumefaciens* to transfer its T-DNA into the plant cell, where it is stably integrated into the nuclear genome, has lead to a widespread use of this organism for gene transfer into plants and plant cells. In order to allow the regeneration of plants after *Agrobacterium tumefaciens* transformation the *onc* genes in the T-region have been deleted, which resulted in a disarmed or non-oncogenic T-DNA. Two types of vector systems have been developed for plant transformation. First a binary system, in which new genes are cloned in between the T-DNA borders of a plasmid containing an artificial T-DNA. This plasmid is subsequently introduced into an *Agrobacterium* strain harbouring a Ti plasmid with an intact *vir* region but lacking the T region (Hoekema *et al*.; Nature 303 (1983) 179-180 and Bevan; Nucl. Acids Res. 12 (1984) 8711-8721). Secondly a co-integrate system, in which new genes are introduced via homologous recombination into an artificial T-DNA already present on a Ti plasmid with an intact *vir* region (Zambryski *et al*.; EMBO-J. 2 (1983) 2143-2150).
A wide variety of plant species have been transformed using such systems. This includes many agriculturally important dicotyledonous species such as potato, tomato, soybean, sunflower, sugarbeet and cotton (for a review see Gasser and Fraley; Science 244, (1989) 1293-1299). Although *Agrobacterium* transformation of monocotyledonous plants seemed to be impossible for a long time, nowadays several species such as maize (Ishida *et al*.; Nature-Biotechnology 14 (1996) 745-750) and rice (Aldemita and Hodges; Planta 199 (1996) 612-617) have been transformed using *Agrobacterium*.
One of the reasons why the method has found wide use in plant transformation is its high transformation frequency. For instance in co-cultivation experiments with tobacco protoplasts about 25% percent of the microcalli, that were regenerated from protoplasts after co-cultivation with *Agrobacterium* (on average 20%), were transformed- (Depicker *et al*.; Mol. Gen. Genet. 201 (1985) 477-484 and Van den Elzen *et al*.; Plant Molecular Biology 5 (1985) 149-154). This means that up to about 5% of the cells are transformed. Furthermore, the method is much easier compared with other plant transformation methods using naked DNA. It is applicable to intact plant tissues such as segments of leaves, stem, root and tubers as well as protoplasts. Additionally, the method has the advantage that only the T-DNA comprising the foreign DNA to be introduced is integrated into the plant genome. The vector DNA sequences required for replication and selection of the vector in the bacterium are not transported from the bacterium to the plant cell. Thus it is a relatively clean transformation method.
Another *Agrobacterium* species, *Agrobacterium rhizogenes*, possesses a similar natural gene transfer system.

### (3) Transformation of micro-organisms using Agrobacterium

In addition to the many publications on transformation of plants using *Agrobacterium tumefaciens*, recently the results of some investigations on the use of *Agrobacterium tumefaciens* for transforming micro-organisms were published. Beijersbergen *et al*. (Science 256 (1992) 1324-1327) demonstrated that the virulence system of *A. tumefaciens* can mediate conjugative transfer between agrobacteria, which only relates to transformation of different strains of the same species.
Bundock *et al*. (EMBO-J. 14 (1995) 3206-3214) reported on successful transformation of yeast by this soil bacterium. This result was subsequently confirmed by Piers *et al*. (Proc. Natl. Acad. Sci. USA, 93 (1996) 1613-1618). Both groups used DNA sequences from *S. cerevisiae* such as the yeast 2µ origin (Bundock *et al*.; EMBO-J. 14 (1995) 3206-3214) or yeast telomeric sequences and the *ARS1* origin of replication (Piers *et al*.; Proc. Natl. Acad. Sci. USA, 93 (1996) 1613-1618) in order to stabilize the T-DNA in yeast. Very recently, Risseeuw *et al*. (Mol. Cell. Biol. 16 (1996) 5924-5932) and Bundock & Hooykaas (Proc. Natl. Acad. Sci. USA, 93 (1996) 15272-15275) reported results on the mechanism of T-DNA integration in *S. cerevisiae*.
The data made available by these publications show that the transformation of micro-organisms by *Agrobacterium tumefaciens* is much less effective than that of plants. As mentioned above, in plants up to about 5% of the cells have been transformed, whereas for yeast much lower ratios of transformed cells / recipient cells are reported, namely 3 x 10⁻³ (Piers *et al*., Proc. Natl. Acad. Sci. USA, 93 (1996) 1613-1618) and 3.3 x 10⁻⁶ (Bundock *et al*. EMBO-J 14 (1995) 3206-3214).

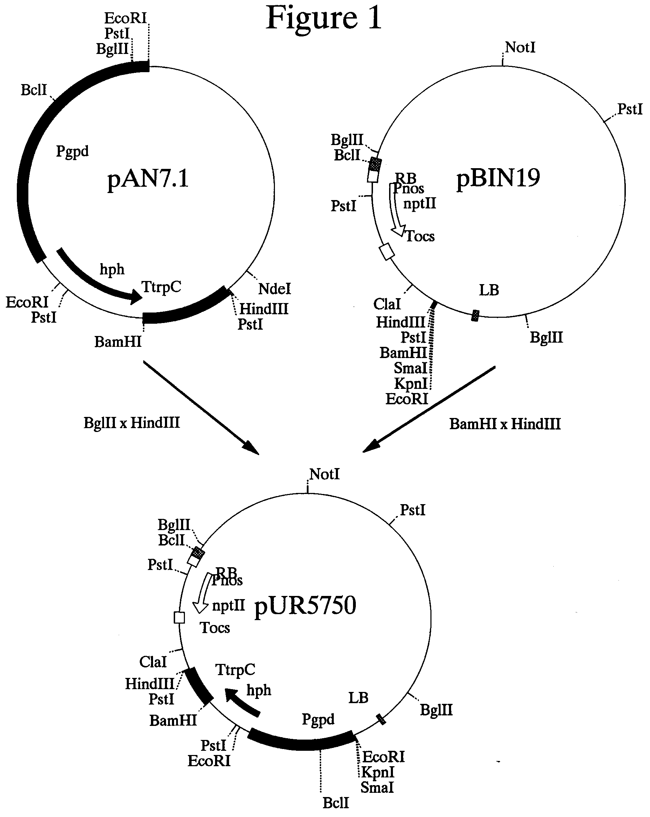

Additionally, *A. tumefaciens* transformation of micro-organisms proved to be less efficient than traditional transformation techniques for micro-organisms. Usually the transformation frequency for naked DNA transfer is depicted as the number of transformants per µg vector DNA, whereas the transformation frequency for *A. tumefaciens* transformation is often expressed as the number of transformed cells that can be obtained in relation to the number of recipient cells. In a prior publication on conventional transformation of yeast (Gietz *et al*.; Yeast 11, (1995) 355-360) both figures on transformants/µg vector DNA and figures on transformed cells per recipient cell are given, which gives a link between the two methods of calculating the transformation frequency. Gietz *et al*. determined that with their LiAc/SS-DNA/PEG procedure a maximum of about 4% of the yeast cells in the reaction could be transformed, i.e. a transformation frequency of up to 4 x 10⁻². From Figure 1A and the corresponding description of this publication one can calculate that this 4% corresponds with 8 x 10⁻⁵ transformants/µg vector DNA. For *A. tumefaciens* transformation of yeast the maximal reported transformation frequencies are 3 x 10⁻³ (Piers *et al*.; Proc. Natl. Acad. Sci. USA, 93 (1996) 1613-1618) and 3.3 x 10⁻⁶(Bundock *et al*.; EMBO-J. 14 (1995) 3206-3214), which is a factor of about 10 or 10,000, respectively, lower than the maximum transformation frequency (4%) of yeast with naked DNA reported by Gietz *et al*.
**Thus based on this evidence *A. tumefaciens* does not seem to be an additional promising tool for the transformation of micro-organisms, because the transformation frequencies obtained with *A. tumefaciens* are much lower than with the conventional transformation methods of yeast.**

### Summary of the invention

The invention is based on the idea of using *Agrobacterium* for transforming moulds. Notwithstanding the just indicated low transformation frequencies obtained with only one yeast species, namely *Saccharomyces cerevisiae*, the inventors decided to investigate the *Agrobacterium tumefaciens* mediated transformation of the mould *Aspergillus awamori*. The latter is an important mould for the production of enzymes, proteins and metabolites, but it has the disadvantage that the conventional mould transformation techniques are relatively inefficient as shown by the figures given above (see Ward *et al*.).

Surprisingly, it was found that the plant transformation technique with *Agrobacterium tumefaciens* could be applied successfully with the mould *Aspergillus awamori*. After some experiments a transformation frequency of more than 7000 transformants per 10⁷ recipient cells was obtained, which is about 400 times the transformation frequency obtained with conventional transformation (see Example 1 below).
Subsequently, this technique was also applied successfully to a wide variety of moulds, including *Aspergillus niger*, *Aspergillus nidulans*, *Fusarium solani pisi* (CBS 230.34), *Fusarium graminearum* (ATCC 20334), *Trichoderma reesei* (CBS 383.78), *Colletotrichum gloeosporioides* (CBS 862.70), *Neurospora crassa* (CBS 195.57), and *Pleurotus ostreatus* (strain Somycel 3015; purchased from "Proefstation voor de Champignoncultuur). These moulds belong to different taxonomic backgrounds as shown in Table 1 below. Table 2 below gives the approximate number of genera and species within each division of the Eumycota. The subdivision Mastigomycotina comprises the Chytridiomycetes and the Oomycetes.

Thus in a broad sense the invention relates to the transformation of moulds, also known as filamentous fungi. The Examples given below represent the three major sub-divisions of the Eumycota which together form about 95% of the mould species (see Table 2).

For *Colletotrichum gloeosporioides* the method of the invention is about 5 to 10 times better than the published frequency for naked DNA transfer (see Example 5). Several of other tested moulds, such as *Fusarium graminearum* (see Example 7), *Neurospora crassa* (see Example 8) *Trichoderma*

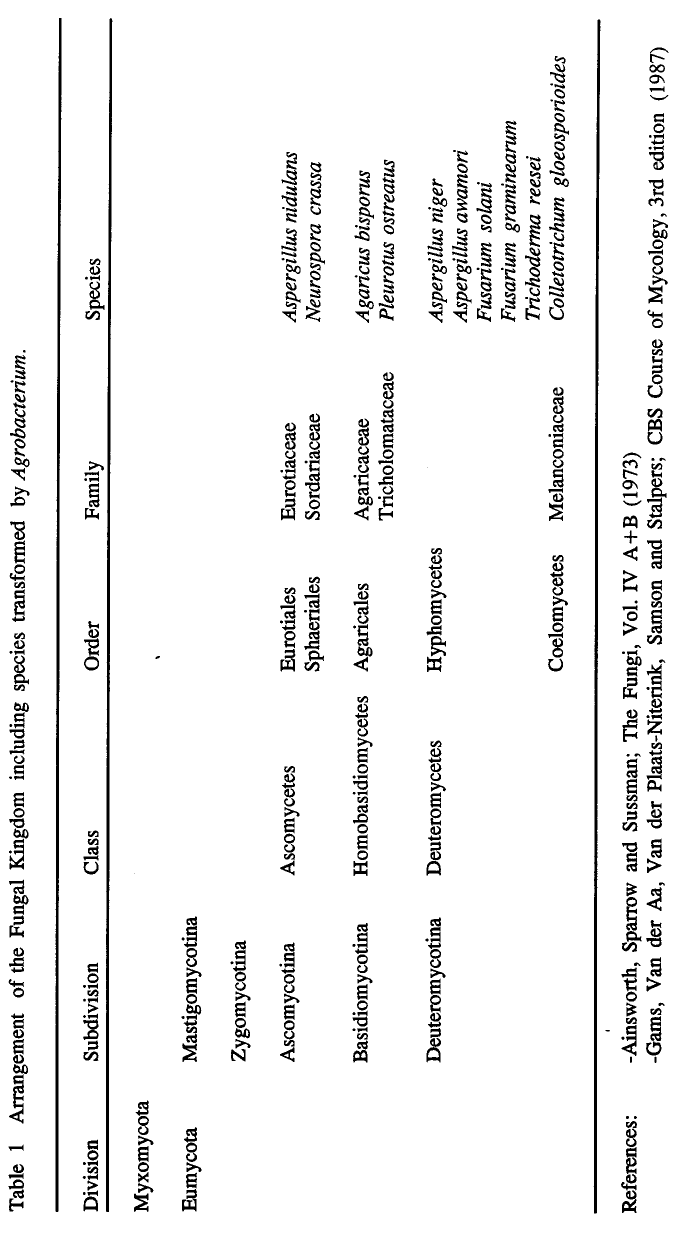

**Table 2**

| Approximate number of genera and species in each division of the Eumycota (as published by O'Donnell and Peterson in Chapter 2 of the book "Biotechnology of Filamentous Fungi, Technology and Products" (1992) 7-33, edited by Finkelstein and Ball). | | |
|---|---|---|
| Division | No. and percentage genera | No. and percentage species |
| Mastigomycotina | 190 ( 3.2) | 1170 ( 1.8) |
| Zygomycotina | 145 ( 2.5) | 765 ( 1.2) |
| Ascomycotina | 2720 (46.6) | 28650 (45.0) |
| Basidiomycotina | 1104 (18.9) | 16000 (25.2) |
| Deuteromycotina | 1680 (28.8) | 17000 (26.8) |

*reesei* (see Example 9), and *Pleurotus ostreatus* (see Example 10), gave transformation frequencies afte*r Agrobacterium* transformation that are similar to the optimal naked DNA transfer methods. The moulds *Aspergillus nidulans*, *Aspergillus niger* and *Fusarium solani* gave transformation frequencies after *Agrobacterium* transformation that are lower than the frequencies for naked DNA transfer. For the *Aspergillus* species this is presumably caused by problems with the selection of transformants (see Examples 3 and 4). It should be noted that transformation of the other moulds has not been optimized. Based on the experience with *Agrobacterium* transformation in plants, it is likely that the transformation frequencies can be increased further.

Many of these moulds are important in industry, agriculture and basic biological research.
For example *Aspergillus awamori*, *Aspergillus niger*, *Trichoderma reesei* and *Fusarium graminearum* have shown to be attractive hosts for large scale production of homologous and heterologous proteins (Van den Hondel et al.; "Heterologous gene expression in filamentous fungi" (Chapter 18) in the book "More Gene Manipulations in Fungi" (1991) 397-428, edited by Bennett and Lasure; Verdoes *et al*.; Appl. Microbiol. Biotechnol 43 (1995) 195-205; Royer *et al*.; Bio/Technology 13 (1995) 1479-1483). They have the capacity to secrete substantial amounts of protein into the medium, large scale fermentation is generally well established and they have a GRAS (Generally Recognized As Safe) status, which makes it possible to use these species in the food and food-processing industry.
Moreover, the mould *Fusarium graminearium* A 3/5, the Quorn^{R} myco-protein fungus, has also been used as a commercial human food source in the UK for over 10 years (Royer *et al*.; Bio/Technology 13 (1995) 1479-1483).
The moulds *Fusarium solani* and *Colletotrichum gloeosporioides* are fungal pathogens (Marek *et al*.; Curr Genet 15 (1989) 421-428; Hwang *et al*.; The Plant Cell 7 (1995) 183-193).
Both *Aspergillus nidulans* and *Neurospora crassa* have been important organisms for basic research into genetic mechanisms, biochemical pathways and cellular physiology (Vollmer and Yanofsky; Proc. Natl. Acad. Sci. USA 83 (1986) 4869-4873; The book "*Aspergillus*: 50 year on" (1994) edited by Martinelli and Kinghorn).
The mushroom *Pleurotus ostreatus* like other mushrooms including *Agaricus bisporus* are edible and commercially important.

### Brief description of the drawings

**Figure 1** shows the construction of plasmid pUR5750. Explanation of the abbreviations used in the construction scheme:
   - RB =: Right T-DNA Border,
   - Pnos =: Promoter sequences of the *nopaline synthase* gene,
   - nptII=: coding region of the *neomycin phosphotransferase* II gene from Tn5,
   - Tocs =: Terminator sequences of the *octopine synthase* gene,
   - TtrpC=: Terminator sequences from the *A. nidulans trpC* gene,
   - hph =: coding region of the *hygromycin phosphotransferas*e gene from *E. coli*,
   - Pgpd =: Promoter sequences of the *A. nidulans gpd* gene,
   - LB =: Left T-DNA Border.

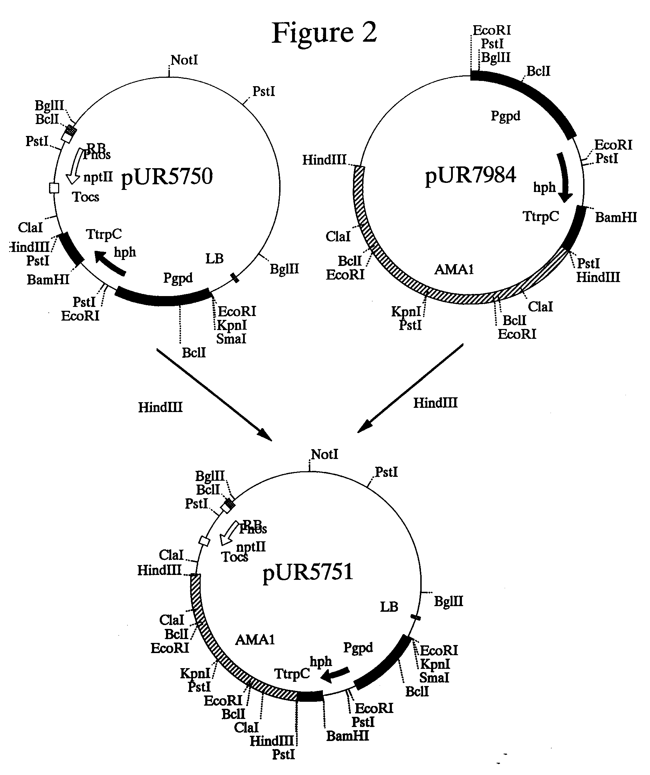

**Figure 2** shows the construction of plasmid pUR5751. Explanation of the abbreviations used in the construction scheme:
   - AMA1 =: the plasmid replicator AMA1 from *Aspergillus nidulans*

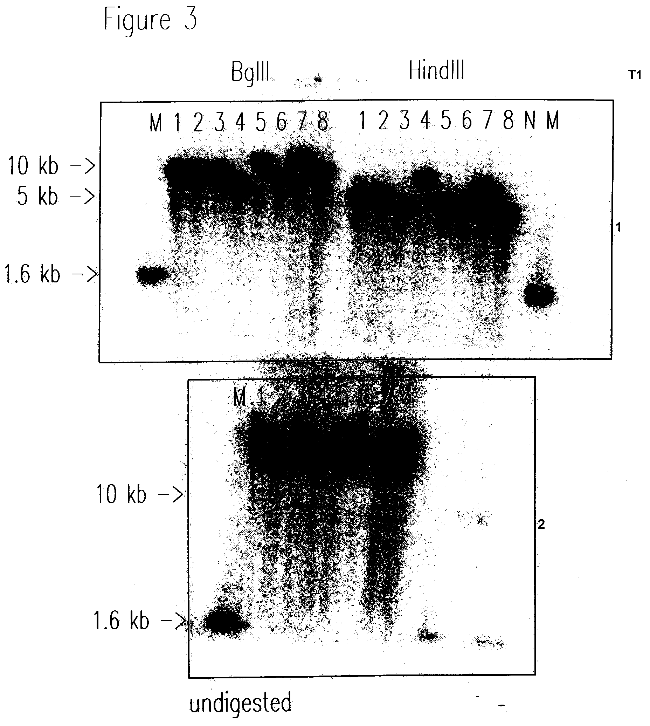

**Figure 3** shows the autoradiograph of the Southern blot of eight independent *Aspergillus awamor*i transformants (nr. 1-8). The genomic DNA was digested with *Bgl*II or *Hind*III (panel 1) or undigested (panel 2). M represents the 1 kb DNA marker (BRL), the hybridizing band represents the 1.6 kb marker fragment. N (in the upper panel) is a negative control sample of non-transformed mould tissue.

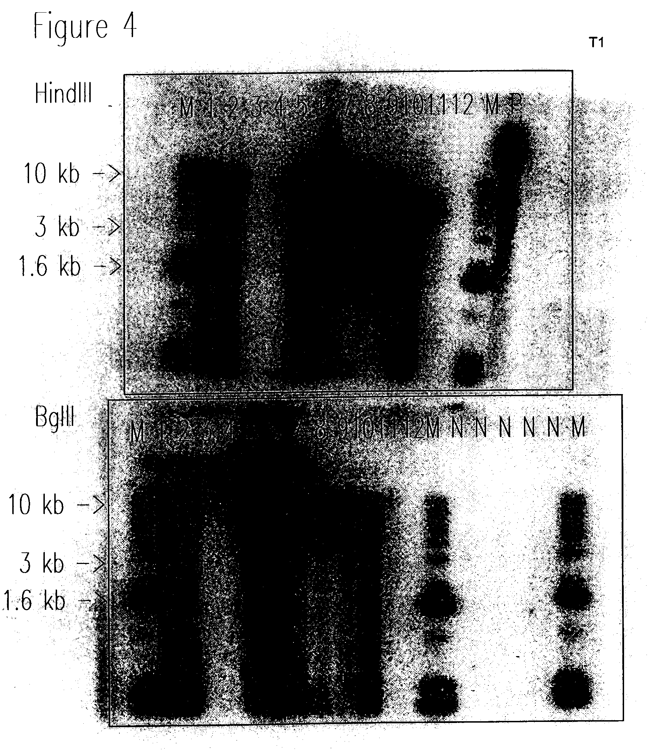

**Figure 4** shows the autoradiograph of the Southern blot of nine independent mould transformants. Number 1 and 2 are *Aspergillus niger* transformants, number 5 and 6 are *Trichoderma reesei* transformants, number 7 and 8 are *Fusarium graminearum* transformants, number 9 and 10 are *Neurospora crassa* transformants and number 11 is a *Colletotrichum gloeosporioides* transformant. The lanes 3, 4 and 12 contained no DNA. The genomic DNA was digested with *Hind*III (upper panel) or *Bgl*II (lower panel). P (in the upper panel) represents the positive control, which is undigested DNA of *Aspergillus awamori* transformant 7 (see figure 3). N (in the lower panel) are negative control samples of non-transformed moulds. M represents the 1 kb DNA marker (BRL), the hybridizing bands represents the 0.5 and 1.6 kb marker fragment.

### Detailed description of the invention

The invention provides a process for producing a transformed mould, characterised in that
(1) a DNA fragment to be introduced into a mould is first cloned into a vector between the T-DNA borders of *Agrobacterium tumefaciens* present in that vector;
(2) the vector containing the DNA fragment between the T-DNA borders is introduced into an *Agrobacterium tumefaciens* strain containing a *vir* region in its DNA;
(3) release of T-DNA containing said DNA fragment from said *Agrobacterium tumefaciens* by addition of a *vir*-inducing compound, and the *Agrobacterium tumefaciens* strain is incubated with the mould to be transformed;
   and
(4) the transformed mould is selected from the untransformed mould depending on the characteristics of the introduced DNA or its expression product, and
   optionally the transformed mould is cultured.

The selection of the transformed mould can be carried out by using a selectable marker. For example, such selectable marker is a characteristic of a naturally-occurring, wild-type mould strain, while the mould strain to be transformed is a mutant strain thereof, deficient in said selectable marker, e.g. the orotidine-5'-phosphate decarboxylase gene (*PyrG* gene) which is present in wild-type *Aspergillus awamori*. Suitable selectable markers include antibiotic resistance markers, genes for utilisation of metabolites not usually utilized in that mould strain, and genes producing an easily-assayable product.
Sometimes the DNA introduced into the mould can be used as the selectable marker. For example, when the introduced DNA is expressed, it can result in a product not produced in the non-transformed mould, but more or less easily assayable. Or the presence or absence of the DNA can be determined by applying PCR techniques.

Preferably the mould belongs to the fungal division of *Eumycota*, more preferably to one of the fungal sub-divisions
- *Ascomycotina* including the species *Aspergillus nidulans* and *Neurospora crassa*,
- *Basidiomycotina* including *Bjerkandera*, *Coprinus*, *Coriolus*, species, and the species *Agaricus bisporus*, *Flammulina velutipes* (Enokitake), *Lentinus edodes* (Shiitake), *Phanerochaete chrysosporium*, *Schizophyllum commune*, *Tricholoma matsutake*, and *Pleurotins ostreatus*,
- *Deuteromycotina* including *Beauveria* and *Metarhizium* species (suitable as biological control agents against insects), *Acremonium* and *Penicillium* species (suitable for production of antibiotics) and the species *Aspergillus niger*, *Aspergillus awamori*, *Fusarium solani*, *Fusarium graminearium*, *Trichoderma reesei*, and *Colletotrichum gloeosporioides*,
- *Mastigomycotina* comprising the Oomycetes including *Achlya* (suitable for production of pharmaceutically active proteins), *Phytophtora*, *Pythium*, and *Plasmopara* species, and the Chytridiomycetes including *Rhizophydium* and *Rhizophlyctis* species, and
- *Zygomycotina* including *Mucor* and *Rhizopus* species.

The invention further provides a transformed mould obtainable by *Agrobacterium* mediated transformation comprising one or more parts of T-DNA border sequences.
Once a transformed mould according to the invention has been obtained, such transformed mould can be used in a process for further culturing.
The invention also provides the further culturing of a transformed mould obtained by a process according to the invention using an *Agrobacterium* mediated transformation.

The use of supervirulent *A. tumefaciens* strains is preferred, because they give a relatively high transformation frequency. Such strains, the use thereof and vectors for making such strains are described in the literature; see Jin *et al*. (J. Bacteriology 169 (1987) 4417-4425 & Molecular Microbiology 7 (1993) 555-562), Raineri *et al*. (BIO/TECHNOLOGY 8 (January 1990) 33-38) and Ishida *et al*. (Nature Biotechnology 14 (1996) 745-750) for plant transformation, and Piers *et al*. (Proc. Natl. Acad. Sci. USA, 93 (1996) 1613-1618) for yeast transformation.
The transformation can be performed by a binary system or by co-integration in a similar way as is known for plant transformation as discussed above in the section on (2) Plant transformation using *Agrobacterium*.
All types of mould tissue can be used including protoplasts, conidio spores, germinating spores, mycelia, and pellets, of which protoplasts, conidia and rehydrated freeze dried culture material are exemplified below.

Advantages of the *Agrobacterium* mediated transformation of moulds include
- it is a "food-grade" method resulting in a mould strain without residues of bacterial antibiotic resistance markers or other bacterial sequences like origins of replication,
- larger parts of DNA can be introduced. In contrast to the older method of naked DNA mould transformation whereby up to about 40 kb DNA can be introduced, with *Agrobacterium* mediated plant transformation at least 150 kb of foreign DNA was introduced into the plant genome (Hamilton *et al*.; Proc. Natl. Acad. Sci. USA 93 (1996) 9975-9979).

### Examples

The invention is exemplified by the following Examples 1-10 preceded by a description of the Materials and Methods that were used. These Examples show the transformation of *A*. *awamori* both protoplasts (Ex. 1) and conidia (Ex. 2), *A*. *nidulans* conidia (Ex. 3), *A*. *niger* conidia (Ex. 4), *Colletotrichum gloeosporioides* (Ex. 5), *Fusarium solani pisi* conidia (Ex. 6), *Fusarium graminearum* both conidia and rehydrated freeze dried ATCC material (Ex. 7), *Neurospora crassa* conidia (Ex. 8), *Trichoderma reesei* conidia (Ex. 9), and *Pleurotus ostreatus* conidia (Ex. 10).

### MATERIALS AND METHODS

### Bacterial and mould strains

For bacterial cloning the *Escherichia coli* strain DH5α (genotype: F⁻, *endA*1, *hsdR*17 (rₖ⁻ mₖ⁺), *supE*44, *thi*-1, lambda⁻, *recA*1, *gyrA*96, *relA*1, Δ(*argF*-*lacIZYA*)U169, *deoR* (phi80d-(*lacz*)ΔM15); Hanahan; J. Mol. Biol. 166 (1983) 557-580) was used. The *Agrobacterium tumefaciens* strain LBA1100 was used for the transformation of moulds (Beijersbergen *et al*., 1992, Science, 256, p. 1324-1327). The mould strains *Aspergillus awamori* #40 (a derivative of *A. awamori* CBS 115.52 also mentioned in WO 93/12237, page 9 line 13), *Aspergillus niger* (strain N402, a cspA1 (short conidiophores) mutant of *Aspergillus niger* var. *niger* ATCC9029, CBS 120.49 described in UNILEVER's WO 91/19782*) Aspergillus nidulans* (Lab collection URL-VL), *Finsarium solani pisi* (CBS 230.34), *Fusarium graminearium* (ATCC 20334), *Trichoderma reesei* (CBS 383.78), *Colletotrichum gloeosporioides* (CBS 862.70), *Neurospora crassa* (CBS 195.57), and *Pleurotus ostreatus* strain Somycel 3015 (purchased from "Proefstation voor de Champignoncultuur", P.O. Box 6042, 5960 AA Horst, The Netherlands), were used as the recipient in transformations with *Agrobacterium tumefaciens*.
The preparation of *A. awamori* #40 (also known as *A. niger* var. *awamori* #40) was described in WO 91/19782 on page 13, lines 29-39, which read:
" The production level of the A. niger var. awamori transformants, however, can be further increased by using suitable A. niger var. awamori mutant strains, such as A. niger var. awamori #40, which produces clearly more xylanase than the wild type strain.
The mutant A. niger var. awamori #40 has been obtained by mutagenesis of A. niger var. awamori spores and selection for xylanase production. In bran medium the "xylA"A. niger var. awamori #40 transformant produced 190 000 U xylanase, which is a considerable increase over the best producing A. niger var. awamori transformant.

In this specification the following endonuclease restriction sites are used:

| giving staggered ends | | giving blunt ends | |
|---|---|---|---|
| *Bam*HI | G↓GATCC | *Sma*I | CCC↓GGG |
| *Bcl*I | T↓GATCA | | |
| *Bgl*II | A↓GATCT | | |
| *Cla*I | AT↓CGAT | | |
| *Eco*RI | G↓AATTC | | |
| *Hin*dIII | A↓AGCTT | | |
| *Kpn*I | GGTAC↓C | | |
| *Not*I | GC↓GGCCGC | | |
| *Pst*I | CTGCA↓G | | |

### Plasmid construction

**Plasmid pUR5750** (see Figure 1) was constructed by cloning a 4 kb *Bgl*II/*Hin*dIII fragment, which is present on the vector **pAN7.1** (Punt *et al*.; Gene 56 (1987) 117-124) and contains the promoter from the *A. nidulans gpd* gene fused to the coding region of the *E. coli hph* gene and followed by terminator sequences from the *A. nidulans trpC* gene, into the *Bam*HI/*Hin*dIII sites of the binary vector **pBIN19** (Bevan, M.; Nucleic Acids Res. 22 (1984) 8711-8721).
**Plasmid pUR5751** (see Figure 2) was constructed by cloning the plasmid replicator AMA1 from *Aspergillus nidulans* (Aleksenko and Clutterbuck; Molecular Microbiology 19 (1996) 565-574) as a 5.3 kb *Hin*dIII fragment from the plasmid **pUR7984** into the *Hin*dIII site of pUR5750. **pUR7984** was obtained by cloning the 5.3 kb AMA1 *Hin*dIII fragment from **pHELP1** (provided by Clutterbuck) into the *Hin*dIII site of pAN7.1. The 5.3 kb AMA1 *Hin*dIII fragment is the fragment between the *Hin*dIII site at position 367 and th*e Hin*dIII site at position 5620 of the sequence deposited in the EMBL/GenBank/DDBJ Nucleotide Sequence Data Library under Ac. no. X78051.
The *Agrobacterium* strain LBA1100, first described by Beijersbergen *et al*. (Science 256 (1992) 1324-1327) and referred to in several later publications, was electroporated with the constructs pUR5750 and pUR5751 according to Mozo and Hooykaas (Plant Mol. Biol. 16 (1991) 917-918).
**This *Agrobacterium* strain LBA1100 has been deposited on 27 March 1997 under the Budapest Treaty at the Centraalbureau voor Schimmelcultures in Baarn, The Netherlands (No. CBS 634.97).**

### Transformation experiments

The *Agrobacterium* strain containing the binary vector pUR5750 was grown at 29°C overnight on LB plates containing the appropriate antibiotics at the following concentrations: kanamycin, 100 µg/ml; spectinomycin, 250 µg/ml; rifampicin, 20 µg/ml. A single colony was streaked on a minimal medium plate. Minimal medium (MM) contains per litre: 10 ml K-buffer pH7.0 (200 g/l K₂HPO₄, 145 g/l KH₂PO₄), 20 ml M-N (30 g/l MgSO₄.7H₂O, 15 g/l NaCl), 1 ml 1% CaCl₃.2H₂O (w/v), 10 ml 20% glucose (w/v), 10 ml 0.01% FeSO₄ (w/v), 5 ml spore elements (100 mg/l ZnSO₄.7H₂O, 100 mg/l CuSO₄.5H₂O, 100 mg/l H₃BO₃, 100 mg/l MnSO₄.H₂O, 100mg/l Na₂MoO₄.2H₂O) and 2.5 ml 20% NH₄NO₃ (w/v) (Hooykaas *et al*.; J. Gen. Microbiol. 110 (1979) 99-109) bacto-agar at 15 g/l and the appropriate antibiotics. The plates were incubated at 29°C for 1 to 2 days. Several colonies were inoculated in minimal medium containing the appropriate antibiotics and grown at 29°C overnight. After dilution of *Agrobacterium* cells to an OD₆₆₀ₙₘ of approximately 0.15 in induction medium the culture was grown for 6 hours at 29°C. The induction medium (IM) differs from minimal medium in that the 10 ml 20% glucose (w/v) was replaced by 10 mM glucose and 40 mM MES (ex Sigma) (pH5.3), 0.5% glycerol (w/v), and 200 µM acetosyringone (AS) were added. In order to confirm that transformation of the moulds by *Agrobacterium* is dependent on T-DNA transfer, a negative control was included in which the *vir* inducer AS was omitted.
Conidia were obtained by growing the mould strains at room temperature on a nitrocellulose filter (Hybond-N, Amersham) placed on a PDA (Potato Dextrose Agar) plate for several days and subsequently washing the filters with physiological salt solution. Protoplasts of *A. awamori* were prepared as described by Punt and Van den Hondel (Methods in Enzymology 216 (1993) 447-457). For transformation of protoplasts, a 100 µl aliquot containing 10⁶ to 10⁷ protoplasts was mixed with 100 µl of the *Agrobacterium* culture. For transformation of conidia, conidia were diluted in physiological salt solution at a concentration of 10⁶, 10⁷ or 10⁸ conidia/ml and 100 µl was mixed with 100 µl of the *Agrobacterium* culture. Subsequently, the mixtures were plated on nitrocellulose filters placed on IM plates (IM medium with 15 g/l bacto-agar) containing 5 mM glucose and incubated at room temperature or 29°C for 2, 3, 5 or 6 days (as indicated in the Examples). The negative control samples were incubated on IM plates in which the *vir* inducer AS was omitted. Hereafter, the filters were transferred to *Aspergillus* minimal medium plates (Bennett and Lasure, Growth media In: Bennett and Lasure (eds) More gene manipulations in fungi, Academic Press, San Diego (1991) 441-458) or PDA plates containing 200 µM cefotaxim to kill the *Agrobacterium* cells and hygromycin (for concentrations see Examples) to select for transformants.

### DNA isolation and Southern analysis

Southern analysis was performed to confirm at a molecular level that the mould cell had been transformed and the desired DNA had been integrated into the genome.
To obtain mycelium material for a genomic DNA isolation, approximately 10⁸ mould conidia were inoculated in 50 ml of *Aspergillus* minimal medium supplemented with 0.5% yeast extract and incubated for a period ranging from 22 hours to 3 days at 30 °C in a shaker at 200 rpm. The mycelium was harvested through Miracloth^{R} (Calbiochem) and snap frozen in liquid N₂. Frozen samples were ground to a fine powder using a Mikro-Dismembrator^{R} (ex Braun Biotech International) for 1 minute at 1750 rpm. Mould genomic DNA was isolated using Qiagen genomic tips (cat. no. 10223) and a protocol for genomic DNA purification from filamentous fungi provided by the supplier. The step for digestion of cell wall material was omitted. Approximately 2.5 µg of DNA was digested with *Bgl*II or *Hin*dIII (4 Units/µg) for 16 hours and separated on a 0.8% agarose TBE gel. DNA was transferred to a Hybond N membrane by capillary blotting (overnight) and the membrane was (pre-)hybridized according to the Hybond protocol.
For the Southern blot presented in Figure 3 the 4 kb *Bgl*II/*Hin*dIII fragment from pAN7.1 described above was used as a probe. For the Southern blot presented in Figure 4 the 0.8 kb *Bam*HI/*Eco*RI fragment from pAN7.1 was used as a probe, which contains part of the *E. coli hph* gene. A DNA probe labelled with α³²P-dCTP was obtained using the RTS RadPrime DNA Labelling System from GibcoBRL (cat. no. 10387-017). The electronic autoradiographs were obtained using an Instant Imager (Packard).

### Example 1 Transformation of A. awamori protoblasts

For protoplast isolation, a shake flask containing 200 ml of MM medium including 0.5% yeast extract was inoculated with 10⁶ conidia/ml of *A. awamori* and incubated for 18 hours at 30°C in a shaker at 200 rpm. Mycelium was harvested through sterile Mirocloth^{R} and washed with ice-cold 0.6 M MgSO₄. The mycelium was resuspended in OM medium (per litre: 500 ml 2.4 M MgSO₄, 480 ml H₂O, 16.8 ml 0.5 M Na₂HPO₄, 3.2 ml 0.5 M NaH₂PO₄, pH 5.8-5.9) at 5 ml/g mycelium. Subsequently, 5 mg Novozym 234^{R} and 6 mg BSA were added per g mycelium. Protoplasting was allowed to proceed for 1-2 hours at 30°C in a shaker at 80-100 rpm. The formation of protoplasts was checked using a light microscope. Protoplasts were filtered through sterile Miracloth^{R} and the sample was divided in 30 ml aliquots in falcon tubes. STC (1.2 M sorbitol, 10 mM Tris/HCl pH 7.5, 50 mM CaCl₂.2H₂O) was added to bring the volume up to 50 ml and the protoplasts were harvested by centrifugation at 2000 rpm for 10 minutes at 4°C. The protoplasts were washed again in 50 ml STC and resuspended in STC at a concentration of approximately 10⁸ protoplasts/ml.
In order to compare the frequency of transformation using *A*. *tumefaciens* with PEG transformation, PEG transformations were also performed. Five µg of pAN7.1 was added to an aliquot of 100 µl (10⁷) protoplasts, mixed and incubated for 25 minutes on ice. PEG was added in two 200 µl aliquots and an 850 µl aliquot, and the mixture was incubated at room temperature for 20 minutes. Finally, the mixture was washed with 10 ml of STC, harvested by centrifugation at 2000 rpm for 10 minutes at room temperature and the sample was plated on a MM plate containing 100 µg/ml hygromycin for selection of transformants.
For *A. tumefaciens* transformation, a 100 µl aliquot containing 3x10⁶ to 10⁷ protoplasts was mixed with 100 µl *A*. *tumefaciens* grown as described in Materials and Methods. From this sample 1/10, 1/100 and 1/1000 dilutions were made in IM. Subsequently, the mixtures were plated on nitrocellulose filters placed on IM plates containing 5 mM glucose and incubated at room temperature or 29°C for 2 days. Hereafter, the filters were transferred to *Aspergillus* MM plates containing 200 µM cefotaxim to kill the *Agrobacterium* cells and 100 µg/ml hygromycin to select for transformants.

Three separate experiments were carried out with *A*. *tumefaciens* containing the binary vector pUR5750. In each experiment transformations were carried out in duplo and a negative control without AS was included. The results are depicted in Table 3 below. Transformed hygromycin resistant cells were only obtained in medium containing AS. The negative controls never gave rise to transformed cells. These results demonstrate unequivocally that induction of the *vir* genes is essential for transfer of the T-DNA to the mould cell and therefore that *A. tumefaciens* is capable of transforming the mould *Aspergillus awamori*. The transformation frequency varied from approximately 300 to 7200 transformants per 10⁷ protoplasts, which is much higher than the values for PEG transformation obtained in earlier non-published experiments. For PEG transformations with pAN7.1 (containing the same expression cassette with the hygromycin gene as selectable marker, which is also present in pUR5750, see Materials and Methods) up to 18 transformants per µg per 10⁷ protoplasts were obtained. This is in agreement with the value of about 12 transformants/µg vector DNA published by Ward *et al*. (see above).
These data demonstrate that by using *A. tumefaciens*-mediated mould transformation up to 400 times more transformants can be generated than with PEG transformation (per µg per 10⁷ protoplasts).
Moreover, in experiment 3 (see Table 3 above) a direct comparison was made between both transformation methods using the same batch of protoplasts. In two PEG transformations, using 5 µg of pAN7.1 per transformation of 10⁷ protoplasts, 6 and 16 transformants were obtained, respectively. On average this is 2.2 transformants per µg per 10⁷ protoplasts. Using *A*. *tumefaciens* transformation 300 and 480 transformants per 10⁷ recipient cells were obtained, thus on average 390 transformants per 10⁷ recipient cells.
**So, by applying a process according to the invention using *A*. *tumefaciens* transformation about 180 times more transformants were obtained.**

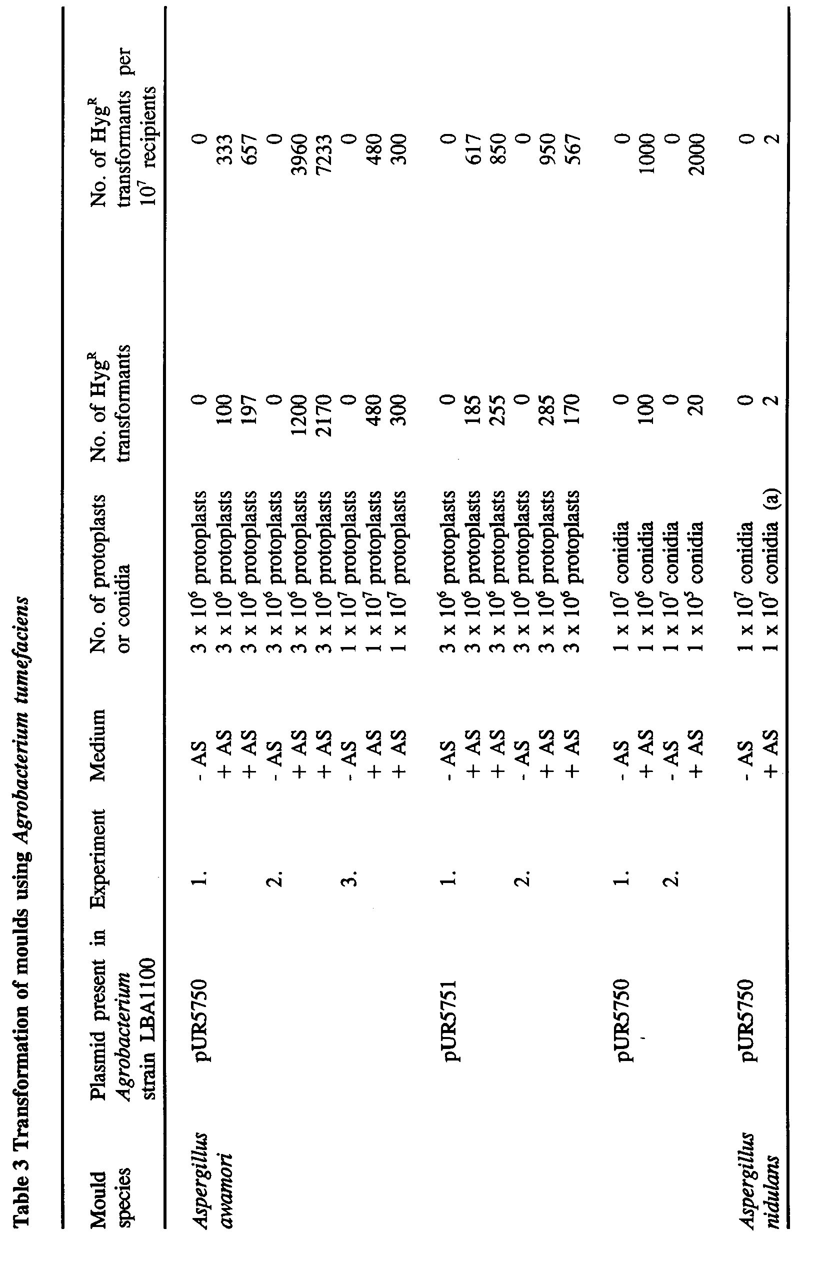

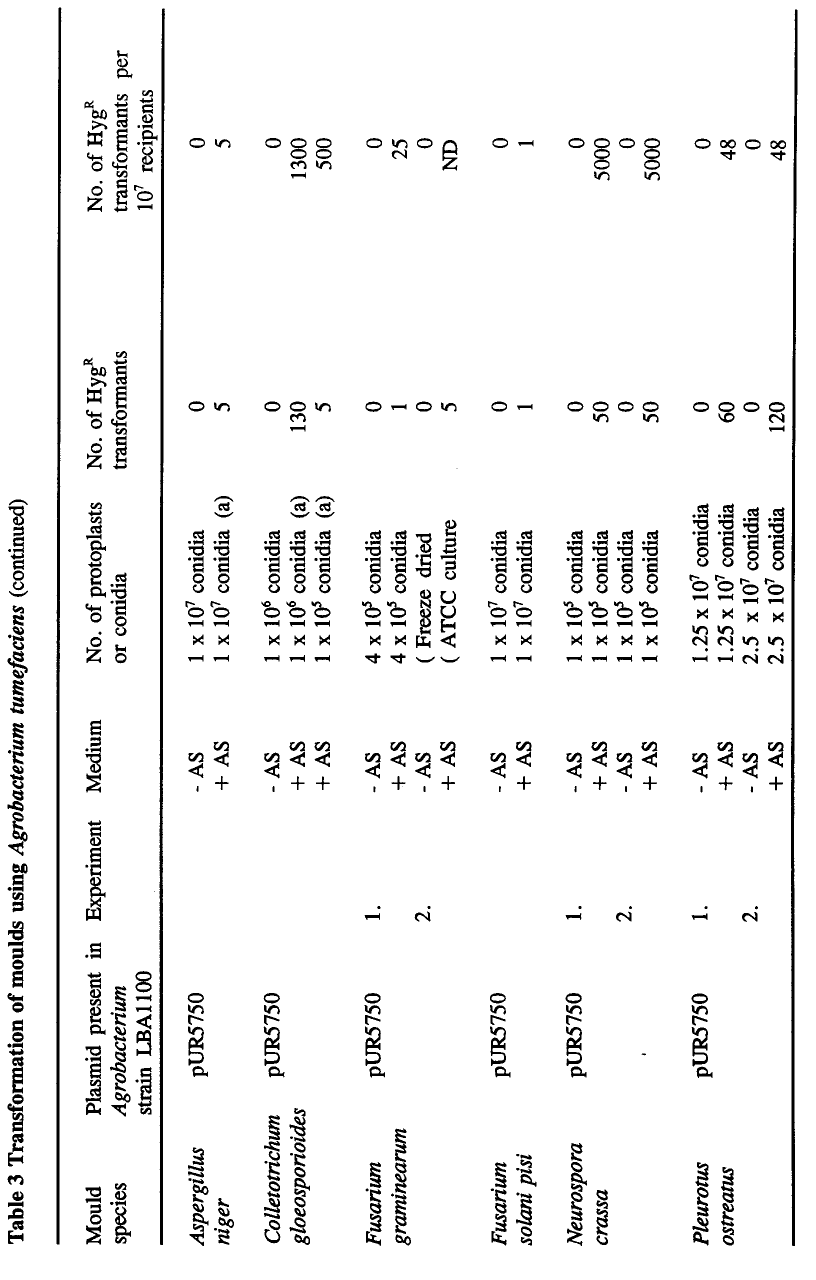

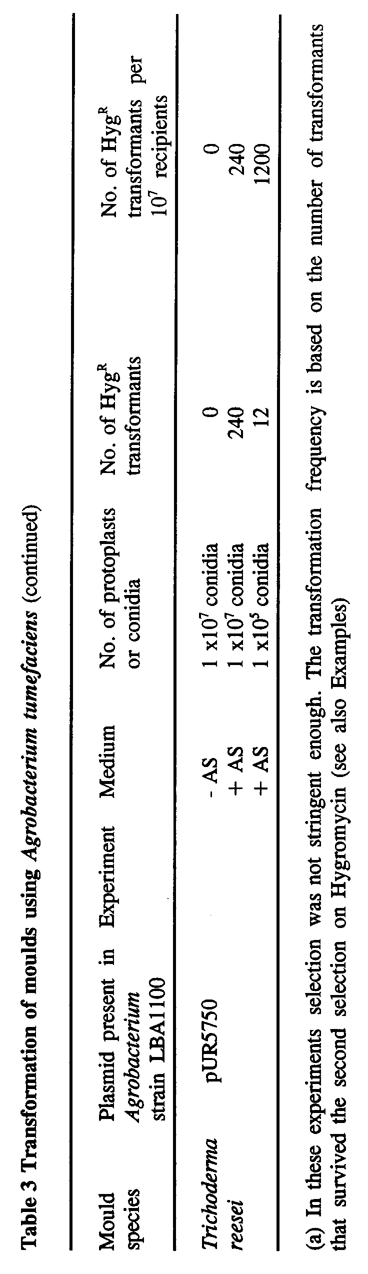

In experiments 1 and 2 the plating efficiency (% surviving cells related to number of starting cells) was determined by plating 1/1000 and 1/10,000 dilutions on MM plates without hygromycin. In experiment 1 the plating efficiency was 5% and in experiment 2 it was 2.6%.
The Hyg resistant phenotype of transformants was confirmed for 78 randomly picked transformants by streaking the conidia on MM plates containing 200 µM cefotaxim and 100 µg/ml hygromycin. From eight of these transformants, conidia from individual colonies were streaked again on MM plates containing 100 µg/ml hygromycin. This was repeated twice. Subsequently conidia were isolated and cultures were grown to obtain mycelium for genomic DNA isolation. DNA isolation and Southern analysis is described in Materials and Methods. The genomic DNA was digested with *Bgl*II or *Hin*dIII. *Bgl*II does not cut within the T-DNA, therefore this digestion will generate a fragment encompassing the whole T-DNA and the chromosomal sequences flanking both the right and left border sites of the T-DNA. This fragment will be at least 7.5 kb. *Hin*dIII cuts once in the T-DNA and the pAN7.1 probe detects only the T-DNA fragment carrying the hygromycin expression cassette and the chromosomal sequences flanking the left T-DNA border. This fragment will be at least 5 kb. Undigested DNA was included in order to confirm the presence of the T-DNA in the high molecular weight chromosomal DNA. The auto-radiographs of the Southern blots are depicted in Figure 3. In all eight transformants the T-DNA was integrated at a single chromosomal locus. Seven out of the eight also contained a single T-DNA integration. In one case the T-DNA was integrated as a tandem repeat. With the undigested DNA samples the hybridization signal coincides with the high molecular weight DNA, which confirms T-DNA integration into the chromosome.

Transformations with *A. tumefaciens* were performed not only with the binary vector pUR5750, but also with the binary vector pUR5751 (see Figure 2). This vector contains the plasmid replicator AMA1 from *Aspergillus nidulans*. Plasmids carrying the AMA1 replicon are capable of autonomous maintenance in *Aspergillus nidulans*. Therefore, this T-DNA should be able to yield a transformed cell wherein the T-DNA is present as an extrachromosomal element. The results of two experiments are depicted in Table 3. The transformation frequency varied from approximately 300 to 950 transformants per 10⁷ protoplasts. The Hyg resistant phenotype of transformants was confirmed for 20 randomly picked transformants by streaking the spores on MM plates containing 100 µg/ml hygromycin.

### Example 2 Transformation of Aspergillus awamori conidia

For *A. tumefaciens* transformation of *Aspergillus awamori* conidia, a 100 µl aliquot containing 10⁷ conidia was mixed with 100 µl *A. tumefaciens* grown as described in Materials and Methods. From this sample 1/10 or 1/100 dilutions were made in IM. Subsequently, the mixtures were plated on nitrocellulose filters placed on IM plates containing 5 mM glucose and incubated at room temperature for 2 days. Hereafter, the filters were transferred to *Aspergillus* MM plates containing 200 µM cefotaxim to kill the *Agrobacterium* cells and 100 µg/ml hygromycin to select for transformants. The results of two experiments are depicted in Table 3 above. Also in this case transformation depended on induction of the *vir* genes by AS. The transformation frequency varied from approximately 1000 to 2000 transformants per 10⁷ conidia, which is in the same range as the frequency after protoplast transformation. The Hyg resistant phenotype of transformants was confirmed for 15 randomly picked transformants by streaking the conidia on MM plates containing 200 µM cefotaxim and 100 µg/ml hygromycin.

### Example 3 Transformation of Aspergillus nidulans conidia

For *A. tumefaciens* transformation of *Aspergillus nidulans* conidia, a 100 µl aliquot containing 10⁷ conidia was mixed with 100 µl *A. tumefaciens* grown as described in Materials and Methods. The mixtures were plated on nitrocellulose filters placed on IM plates containing 5 mM glucose and incubated at room temperature for 2 days. Hereafter, the filters were transferred to *Aspergillus* minimal medium plates containing 200 µM cefotaxim to kill the *Agrobacterium* cells and 1000 µg/ml hygromycin to select for transformants. The filter was overlaid with MM agar containing cefotaxim and hygromycin at the same concentrations.

With *Aspergillus nidulans* selection proved to be cumbersome. Apparently, during co-cultivation the conidia germinated and grew out too far to allow a stringent selection. This observation is in accordance with results obtained by Cullen et al. (Gene 57 (1989) 21-26). They determined that the incubation time before starting selection is very important for a good result. An incubation period of more than 16 hours before selection was applied, resulted in significant background growth, whereas after such incubation period of only 8 hours no colonies were observed. The reported figures were obtained after such incubation period of 12 hours. They also observed a substantial strain variability with respect to hygromycin sensitivity. In view of the results of Cullen *et al*. the transformation frequency of this Example may be improved by optimising the incubation period before applying the selection.
The result is depicted in Table 3 above. In total 15 putative transformed colonies were obtained. Moreover, the negative control yielded three growing and sporulating colonies. In order to confirm the transformed phenotype, conidia were streaked on MM plates containing 200 µM cefotaxim and 1000 µg/ml hygromycin. The negative controls did not grow and only 2 out of the 15 putative transformants could grow. Therefore, also in this case transformation depended on induction of the *vir* genes by AS.

Literature data on PEG transformations using the hygromycin resistance gene show transformation frequencies of 5-20 transformants per µg vector DNA (Cullen *et al*.; Gene 57, (1989) 21-26; Punt *et al*.;, Gene 56 (1987) 117-124).
Using another selectable marker, the *arg*B gene, Fungaro *et al*. (FEMS Microbiology Letters 125, (1995) 293-298) obtained up to 81 transformants per µg vector DNA, whereas 20-300 transformants per µg vector DNA were obtained with the *trpC* gene as the marker (Yelton *et al*. Proc. Natl. Acad. Sci. USA 81 (1984) 1470-1474). These literature data suggest that the number of transformants, that can be obtained using *Agrobacterium* transformation, can be improved by using another selectable marker gene.

### Example 4 Transformation of Aspergillus niger conidia

For *A. tumefaciens* transformation of *Aspergillus niger* conidia, a 100 µl aliquot containing 10⁵, 10⁶ or 10⁷ conidia was mixed with 100 µl *A. tumefaciens* grown as described in Materials and Methods. The mixtures were plated on nitrocellulose filters placed on IM plates containing 5 mM glucose and incubated at room temperature for 2 days. Hereafter, the filters were transferred to *Aspergillus* minimal medium plates containing 200 µM cefotaxim to kill the *Agrobacterium* cells and 200 µg/ml hygromycin to select for transformants. In general, selection proved to be cumbersome. Apparently, during co-cultivation the conidia germinated and grew out too far to allow a stringent selection. This could be improved to some extent by using an overlay on the filter consisting of MM agar containing 200 µM cefotaxim and 200 µg/ml hygromycin.
The results of a typical experiment are depicted in Table 3. The experiment yielded 6 growing colonies on the negative control plate and 6 putative transformed colonies on the transformation plates containing AS. In order to confirm the Hyg resistant phenotype of these colonies, conidia from all twelve colonies were streaked on MM plates containing 200 µM cefotaxim and 200 µg/ml hygromycin. Only five out of the six putative transformants grew on the new selection plates. The remaining putative transformant and the colonies from the negative control experiment did not grow. Therefore also in this case transformation depended on induction of the *vir* genes by AS. Two transformants were subjected to Southern analysis. The genomic DNA was digested with *Bgl*II or *Hin*dIII. *Bgl*II does not cut within the T-DNA, therefore this digestion will generate a fragment encompassing the whole T-DNA and the chromosomal sequences flanking both the right and left border sites of the T-DNA. This fragment will be at least 7.5 kb. *Hin*dIII cuts once in the T-DNA and the probe for the *hph* gene from pAN7.1 detects only the T-DNA fragment carrying the hygromycin expression cassette and the chromosomal sequences flanking the left T-DNA border. This fragment will be at least 5 kb. The Southern analysis (see Figure 4) demonstrated that in both transformants the T-DNA was integrated at a single chromosomal locus, which confirmed the transformed phenotype at the molecular level.

Literature data on PEG-mediated protoplast transformations using the hygromycin resistance gene show transformation frequencies of 5-20 transformants per µg (Punt *et al*.; Gene 56 (1987) 117-124) and up to 17,000 transformants per µg (Mohr and Esser; Appl Microbiol Biotechnol 34 (1990) 63-70). However, the latter authors mention in their publication:
" After colony purification of primary isolates on complete medium, only a few strains (about 10%) grew on hygromycin medium. "
Apparently, they had problems with their selection method. For transformation of intact germinating conidia by electroporation using the *argB* gene, a transformation frequency of 0.5-4 transformants per µg is described (Ozeki *et al*.; Biosci. Biotech. Biochem. 58 (1994) 2224-2227).

### Example 5 Transformation of Colletotrichum gloeosporioides conidia

For *A. tumefaciens* transformation of *Colletotrichum gloeosporioides* conidia, a 100 µl aliquot containing 10⁵ or 10⁶ conidia was mixed with 100 µl *A. tumefaciens* grown as described in Materials and Methods. The mixtures were plated on nitrocellulose filters placed on IM plates containing 5 mM glucose and incubated at room temperature for 2 days. Hereafter, the filters were transferred to *Aspergillus* minimal medium plates containing 200 µM cefotaxim to kill the *Agrobacterium* cells and 100 µg/ml hygromycin to select for transformants. After five days incubation at room temperature colonies appeared on the transformation plates.
The transformation with 10⁵ conidia gave 7 transformants, whereas the transformation with 10⁶ conidia gave 175 transformants. No colonies were obtained on the negative control plate. One day later small colonies started to appear on the negative control plate. Apparently, selection was not tight enough to inhibit the growth of non-transformed cells completely. In order to confirm the Hyg resistant phenotype, mycelium from 11 putative transformed colonies and 3 colonies from the negative control was transferred onto MM plates containing 200 µM cefotaxim and 100 µg/ml hygromycin. Eight out of the eleven putative transformants grew on the new selection plates. The three remaining putative transformants and the three colonies from the negative control experiment did not grow. Therefore also in this case transformation depended on induction of the *vir* genes by AS. The results depicted in Table 3 are corrected for the false positives that were obtained. Transformation yielded 500 to 1300 transformants per 10⁷ conidia. One transformants was subjected to Southern analysis as described in Example 4. This demonstrated that the T-DNA was integrated at a single chromosomal locus, which confirmed the transformed phenotype at the molecular level.

Stephenson *et al*. (Aust. Soc. Biochem. Mol. Biol. 26 (1994) Pos-1-31) reported a transformation frequency of less than 100 transformants per µg. Previously, Armstrong and Harris (Phytopathology 83 (1993) 328-332) had reported a transformation frequency of 2-50 transformants per 10⁸ protoplasts when they used benomyl fungicide resistance for selection.

### Example 6 Transformation of Fusarium solani pisi conidia

For *A. tumefaciens* transformation of *Fusarium solani pisi* conidia, a 100 µl aliquot containing 10⁵ or 10⁷ conidia was mixed with 100 µl *A. tumefaciens* grown as described in Materials and Methods. The mixtures were plated on nitrocellulose filters placed on IM plates containing 5 mM glucose and incubated at room temperature for 2 days. Hereafter, the filters were transferred to *Aspergillus* minimal medium plates containing 200 µM cefotaxim to kill the *Agrobacterium* cells and 100 µg/ml hygromycin to select for transformants. The results are depicted in Table 3.

Transformation of 10⁷ conidia gave 1 transformant. No colonies were obtained on the negative control plate. The Hyg resistant phenotype of the transformant was confirmed by growing the transformant on MM plates containing 200 µM cefotaxim and 100 µg/ml hygromycin. Also in this case transformation depended on induction of the *vir* genes by AS.

When the hygromycin resistance gene was used for PEG transformations of protoplasts, transformation frequencies of 10,000 transformants per µg per 10⁷ protoplasts have been reported for *Fusarium solani* f.sp. *cucurbitae* race 2 (Crowhurst *et al*. Current Genetics 21 (1992) 463-469). Transformation of *Finsarium solani* f.sp. *phaseoli* by PEG and lithium acetate, as reported by Marek *et al*. (Curr. Genet. 15 (1989) 421-428), yielded 0.2 to 3.3 transformants per µg.

### Example 7 Transformation of Fusarium graminearum conidia and rehydrated freeze dried ATCC material

For *A. tumefaciens* transformation of *Fusarium graminearum* conidia, a 100 µl aliquot containing 4 x 10⁵ conidia was mixed with 100 µl *A. tumefaciens* grown as described in Materials and Methods. Also a rehydrated freeze-dried culture obtained from the American Type Culture Collection was used for transformation. The freeze-dried material, present in a double vial, was rehydrated by the addition of 0.4 ml of sterile water and incubated for 30 minutes at RT. The material that was used for transformation has been stored at 4°C for approximately two weeks. An aliquot of 100 µl ATCC material was mixed with 200 µl *A. tumefaciens*. The mixtures were plated on nitrocellulose filters placed on IM plates containing 5 mM glucose and incubated at room temperature for 2 days. Half of the ATCC material was co-cultivated on IM plates for five days. Hereafter, the filters were transferred to PDA plates containing 200 µM cefotaxim to kill the *Agrobacterium* cells and 150 µg/ml hygromycin to select for transformants.
The results are depicted in Table 3. Transformation of 4 x 10⁵ conidia gave 1 transformant, which is 25 transformants per 10⁷ conidia. For the ATCC material five transformants were obtained when it had been co-cultivated for 5 days. No colonies were obtained on the negative control plate. The Hyg-resistant phenotype of the transformant was confirmed by growing the transformants on PDA plates containing 200 µM cefotaxim and 150 µg/ml hygromycin. Also in this case transformation depended on induction of the *vir* genes by AS. Two transformants obtained after transformation of the ATCC material were subjected to Southern analysis as described in Example 4. This demonstrated that the T-DNA was integrated at a single chromosomal locus, which confirmed the transformed phenotype at the molecular level.

Transformation of 5 x 10⁶ to 2 x 10⁷ protoplasts of *Fusarium graminearum* with the *A. nidulans* acetamidase gene using a PEG transformation method, resulted in transformation frequencies of 5 transformants per µg (Royer *et al*., Bio/technology 13 (1995), p. 1479-1483).

### Example 8 Transformation of Neurospora crassa conidia

For *A. tumefaciens* transformation of *Neurospora crassa* conidia, a 100 µl aliquot containing 10⁵ conidia was mixed with 100 µl *A. tumefaciens* grown as described in Materials and Methods. The mixtures were plated on nitrocellulose filters placed on IM plates containing 5 mM glucose and incubated at room temperature for 2 days. Hereafter, the filters were transferred to *Aspergillus* minimal medium plates containing 200 µM cefotaxim to kill the *Agrobacterium* cells and 200 µg/ml hygromycin to select for transformants.
The results are depicted in Table 3. In the first experiment transformation of 10⁵ conidia gave approximately 50 transformants, whereas the 1/10 dilution gave 5 transformants. In the second experiment transformation of 10⁵ conidia gave also approximately 50 transformants. This means that transformation gives up to 5000 transformants per 10⁷ conidia. The Hyg resistant phenotype of 20 transformants was confirmed by growing the transformants on *Aspergillus* minimal medium plates containing 200 µM cefotaxim and 200 µg/ml hygromycin. Also in this case transformation depended on induction of the *vir* genes by AS. Two transformants were subjected to Southern analysis as described in Example 4. This demonstrated that the T-DNA was integrated at a single chromosomal locus, which confirmed the transformed phenotype at the molecular level.

*Neurospora crassa* has been transformed using a variety of methods. Germinating conidia have been transformed by electroporation using the hygromycin resistance gene, transformation frequencies of 3000 to 6000 transformants per µg per 10⁷ conidia were obtained (Chakraborty *et al*.; Can. J. Microbiol. 37 (1991) 858-863. Lithium acetate transformations of germinating conidia resulted in transformation frequencies of 2 to 10 transformants per µg per 10⁷ conidia (Dhawale *et al*.; Curr. Gen. 8 (1984) 77-79). PEG transformation of protoplasts resulted in transformation frequencies ranging from 400 to 15.000 transformants per µg (Vollmer and Yanofsky; Proc. Natl. Acad. Sci. USA 83 (1986) 4867-4873).

### Example 9 Transformation of Trichoderma reesei conidia

For *A. tumefaciens* transformation o*f Trichoderma reesei* conidia, a 100 µl aliquot containing 10⁵ or 10⁷ conidia was mixed with 100 µl *A. tumefaciens* grown as described in Materials and Methods. The mixtures were plated on nitrocellulose filters placed on IM plates containing 5 mM glucose and incubated at room temperature for 2 days. Hereafter, the filters were transferred to *Aspergillus* minimal medium plates containing 200 µM cefotaxim to kill the *Agrobacterium* cells and 100 µg/ml hygromycin to select for transformants.
The results are depicted in Table 3. Transformation of 10⁷ conidia gave approximately 240 transformants, whereas transformation of 10⁵ conidia gave 12 transformants. This means that the transformation frequency varies between 240 and 1200 transformants per 10⁷ conidia. The Hyg resistant phenotype of 9 transformants was confirmed by growing the transformants on *Aspergillus* minimal medium plates containing 200 µM cefotaxim and 100 µg/ml hygromycin. Also in this case transformation depended on induction of the *vir* genes by AS.

Two transformants were subjected to Southern analysis as described in Example 4. This demonstrated that the T-DNA was integrated at a single chromosomal locus, which confirmed the transformed phenotype at the molecular level.

When the same hygromycin selectable marker gene (pAN7.1) is used for PEG transformation of protoplasts, approximately 100 transformants per µg per 10⁷ protoplasts were obtained (Mach *et al*.; Current Genetics 25 (1994) 567-570). When the hygromycin gene was flanked by homologous expression signals, derived from *Trichoderma reesei* itself, Mach *et al*. reported increased transformation frequencies of 1800 to 2500 transformants per µg per 10⁷ protoplasts. Similar results were reported by Gruber (Curr. Genet. 18 (1990) 447-451). Using heterologous vectors they obtained about 800 to 1500 transformants per µg. A vector containing the homologous *pyrG* gene yielded up to 12.000 transformants per µg.

### Example 10 Transformation of Pleurotus ostreatus conidia

Few publications are known on the transformation of edible mushrooms. Peng *et al*. (Curr. Genet. 22 (1992) 53-59) succeeded in transforming *Pleurotus ostreatus*, but the transformed strains were unstable. However, recently Yanai *et al*. (Biosci. Biotech. Biochem. 60 (1996) 472-475) obtained stable transformants of *Pleurotis ostreatus*.
Another cultivated mushroom *Agaricus bisporus* has recently been transformed by Van Rhee *et al*. (Mol Gen Genet 250 (1996) 252-258).

For *A. tumefaciens* transformation of *Pleurotus ostreatus* conidia, two procedures have been used. In experiment 1 an aliquot of 1.25 x 10⁷ conidia was mixed with 150 µl *A. tumefaciens* grown as described in Materials and Methods. The mixture was plated on a nitrocellulose filter placed on an IM plate containing 5 mM glucose. In experiment two 2.5 x 10⁷ conidia were plated on a nitrocellulose filter placed on a PDA plate and pre-incubated for 4 days at room temperature. Subsequently, the filters were transferred to a petridish, submerged in 25 ml of *Agrobacterium* culture in IM (grown for 6 hours as described in Materials and Methods) and incubated for 1 hour at room temperature. Hereafter, the filter was placed on an IM plate containing 5 mM glucose. The plates were incubated at room temperature for 3 or 6 days. Hereafter, the filters were transferred to PDA plates containing 200 µM cefotaxim to kill the *Agrobacterium* cells and 75 µg/ml hygromycin to select for transformants.
The results after 6 days co-cultivation are depicted in Table 3. Transformation of 10⁷ conidia resulted in approximately 48 transformants.
When conidia were used directly for transformation, transformants were only obtained after 6 day co-cultivation (experiment 1). However, when the conidia had been pre-incubated for 4 days prior to transformation (experiment 2), transformants were obtained after 3 and 6 days of co-cultivation, although after 3 days the number of transformants was lower than after 6 days: 10 instead of 48. Also in this case transformation depended on induction of the *vir* genes by AS.

When the same hygromycin selectable marker gene (pAN7.1) is used for PEG transformation of protoplasts, approximately 5-46 transformants per µg per 10⁷ viable protoplasts were obtained (Peng *et al*.; Current Genetics 22 (1992) 53-59). Using bialaphos as a dominant selectable marker Yanai *et al*. (Biosci. Biotech. Biochem. 60 (1996) 472-475) obtained about 2 transformants per µg.

### REFERENCES

Ainsworth, Sparrow and Sussman; The Fungi vol IVA+B (1973)
Aldemita and Hodges,; Planta 199 (1996) 612-617
Aleksenko and Clutterbuck; Molecular Microbiology 19 (1996) 565-574
Armstrong and Harris; Phytopathology 83 (1993) 328-332
Beijersbergen *et al*.; Science 256 (1992) 1324-1327
Bennett and Lasure, Growth media In: Bennett and Lasure (eds) More gene manipulations in fungi, Academic Press, San Diego (1991) 441-458
Bevan; Nucl. Acids Res. 12 (1984) 8711-8721
Bundock *et al*.; EMBO-Journal 14 (1995) 3206-3214
Bundock & Hooykaas; Proc. Natl. Acad. Sci. USA, 93 (1996) 15272-15275
Chakraborty *et al*. Can. J. Microbiol. 37 (1991) p. 858-863
Chassy and Flickinger; FEMS Microbiology Letters 44 (1987) 173-177
Crowhurst *et al*. Current Genetics 21 (1992) 463-469
Cullen *et al*.; Gene 57, (1989) 21-26
Depicker *et al*.; Mol. Gen. Genet. 201 (1985) 477-484
Van den Elzen *et al*.; Plant Molecular Biology 5 (1985) 149-154
Dhawale *et al*. Curr. Gen. 8 (1984) p. 77-79
Dower *et al*.; Nucleic Acids Research 16 (1988) 6127-6145
Fincham, J.R.S.; "Transformation in Fungi" published in Microbio-logical Reviews (Mar. 1989) 148-170
Finkelstein, D.B.; "Transformation" (Chapter 6) in the book "Biotechnology of Filamentous Fungi, Technology and Products" (1992) 113-156, edited by Finkelstein and Ball
Fungaro *et al*.; FEMS Microbiology Letters 125, (1995) 293-298
Gams, Van der Aa, Van der Plaats-Niterink, Samson and Stalpers; CBS Course of Mycology 3rd edition (1987)
Gasser and Fraley; Science 244, (1989) 1293-1299
Gietz *et al*.; Yeast 11 (1995) 355-360
Gruber; Curr. Genet. 18 (1990) 447-451
Hamilton *et al*.; *Proc. Natl. Acad. Sci. USA* 93 (1996) 9975-9979
Hanahan, D., 1983, J. Mol. Biol. 166, p.557-580
Herzog *et al*.; Appl. Microbiol. Biotechnol. 45 (1996) 333-337
Hoekema *et al*.,; Nature 303 (1983) 179-180
Hooykaas *et al*.; J. Gen. Microbiol. 110 (1979) 99-109
Hooykaas and Beijersbergen; Annu. Rev. Phytopathol. 32 (1994) 157-179
Hooykaas and Schilperoort; Plant Molecular Biology 19 (1992) 15-38
Hwang *et al*.; The Plant Cell 7 (1995) 183-193
Ishida *et al*.; Nature-Biotechnology 14 (1996) 745-750
Jin *et al*.; J. Bacteriology 169 (1987) 4417-4425
Jin *et al*.; Molecular Microbiology 7 (1993) 555-562
Lorito *et al*.; Curr. Genet. 24 (1993) 349-356
Mach *et al*.; Current Genetics 25 (1994) 567-570
Marek *et al*.; Curr. Genet. 15 (1989) 421-428
Martinelli and Kinghorn; The book "*Aspergillus*: 50 year on" (1994)
Meilhoc *et al*.; Bio/Technology 8 (1990) 223-227
Miller *et al*.; Proc. Natl. Acad. Sci. USA 85 (1988) 856-860
Mohr and Esser; Appl Microbiol Biotechnol 34 (1990) 63-70
Mozo and Hooykaas; Plant Mol. Biol. 16 (1991) 917-918
O'Donnell and Peterson; Chapter 2 in the book "Biotechnology of Filamentous Fungi, Technology and Products" (1992) 7-33, edited by Finkelstein and Ball
Ozeki *et al*.; Biosci. Biotech. Biochem. 58 (1994) 2224-2227
Peng *et al*.; Curr. Genet. 22 (1992) 53-59
Piers *et al*.; Proc. Natl. Acad. Sci. USA, 93 (1996) 1613-1618
Punt *et al*.; Gene 56 (1987) 117-124
Punt and Van den Hondel (Methods in Enzymology 216 (1993) 447-457
Raineri *et al*.; BIO/TECHNOLOGY 8 (January 1990) 33-38
Risseeuw *et al*.; Mol. Cell. Biol. 16 (1996) 5924-5932
Royer *et al*.; Bio/Technology 13 (1995) 1479-1483
Stephenson *et al*.; Aust. Soc. Biochem. Mol. Biol. 26 (1994) Pos-1-31
Timberlake, W.E. and Marshall, M.A.; Genetic engineering of filamentous fungi; Science 244 (1989) 1313-1317.
Van den Hondel *et al*.; "Heterologous gene expression in filamentous fungi" (Chapter 18) in the book "More Gene Manipulations in Fungi" (1991) 397-428, edited by Bennett and Lasure
Van Rhee *et al*.; Mol Gen Genet 250 (1996) 252-258
Van den Elzen *et al*.; Plant Molecular Biology 5 (1985) 149-154
Verdoes *et al*.; Appl. Microbiol. Biotechnol. 43 (1995) 195-205
Volmer and Yanofsky; Proc. Natl. Acad. Sci. USA 83 (1986) 4869-4873
Ward *et al*.; Experimental Mycology 13 (1989) 289-293
Yanai *et al*. (Biosci. Biotech. Biochem. 60 (1996) 472-475)
Yelton *et al*.; Proc. Natl. Acad. Sci. USA 81 (1984) 1470-1474
Zambryski *et al*.; EMBO-J. 2 (1983) 2143-2150

Information on a deposit of a micro-organism under the Budapest Treaty is given above on page 16, lines 26-29. In agreement with Rule 28 (4) EPC, or a similar arrangement for a State not being a Contracting State of the EPC, it is hereby requested that a sample of such deposit, when requested, will be submitted to an expert only.

## Claims

1. A process for producing a transformed mould, characterised in that
(1) a DNA fragment to be introduced into a mould is first cloned into a vector between the T-DNA borders of *Agrobacterium tumefaciens* present in that vector;
(2) the vector containing the DNA fragment between the T-DNA borders is introduced into an *Agrobacterium tumefaciens* strain containing a *vir* region in its DNA;
(3) release of T-DNA containing said DNA fragment from said *Agrobacterium tumefaciens* by addition of a *vir*-inducing compound, and the *Agrobacterium tumefaciens* strain is incubated with the mould to be transformed;
and
(4) the transformed mould is selected from the untransformed mould depending on the characteristics of the introduced DNA or its expression product, and
optionally the transformed mould is cultured.

2. A process according to claim 1, in which the mould belongs to the group of *Eumycota.*

3. A process according to claim 1, in which the mould is selected from the group consisting of the fungal subdivisions *Ascomycotina*, *Basidiomycotina*, *Deuteromycotina*, *Mastigomycotina*, and *Zygomycotina*.

4. A transformed mould obtainable by *Agrobacterium* mediated transformation comprising one or more parts of T-DNA border sequences.

5. A process for culturing a transformed mould obtained by a process as claimed in any one of claims 1-3.

6. A process for culturing a transformed mould as claimed in claim 4.
